# EUROPEAN PATENT SPECIFICATION

(11) **EP 4 192 540 B1**
(45) Date of publication and mention of the grant of the patent: **25.02.2026**
(21) Application number: 21762794.2
(22) Date of filing: 02.08.2021
(51) Int. Cl.: A61M 1/00, A61M 39/24

(54) **NEGATIVE PRESSURE MEDICAL DEVICE**
MEDIZINISCHE VORRICHTUNG MIT NEGATIVEM DRUCK
DISPOSITIF MÉDICAL À PRESSION NÉGATIVE

(30) Priority: 06.08.2020 IT 202000019474
(43) Date of publication of application: 14.06.2023
(73) Proprietor: Medicud S.r.l., 00197 Rome (IT)
(72) Inventor: D'ANGELO, Antonio Nunzio, 00197 Rome (IT); CAZZULANI, Andrea, 00197 Rome (IT)
(74) Representative: Pace Napoleone, Maria
(86) International application number: PCT/IB2021/057056
(87) International publication number: WO 2022/029603

(56) References cited:
- WO-A2-2015/003194
- US-A1- 2014 188 061
- US-A1- 2016 015 592
- US-A1- 2018 353 660
- US-B1- 6 174 306

## Description

### TECHNICAL FIELD

The present invention relates to a medical device for treating wounds resulting from metabolic diseases such as diabetes or for treating any surgical incision. In particular, the present invention relates to a negative pressure device.

### BACKGROUND ART

The inflammation process which occurs during wound healing is characterized by excessive blood flow to the damaged tissue. Platelets tend to aggregate to form fibroblasts and new tissue while white blood cells and red blood cells represent the so-called exudate. If the wound is exposed to microorganisms or other external pathogens, an infection of the wound could develop, thus causing a worsening of the patient's condition and inevitably a lengthening of the healing times. Thus, the elimination of these pathogens from the damaged tissue accelerates and improves the wound healing process.

The use of negative pressure devices to treat wounds of various kinds has spread considerably over the last thirty years in combination with the use of electric vacuum generators. Negative pressure wound therapy has been shown to be extremely effective in treating acute, chronic or exudative wounds such as ulcers (pressure, diabetic or venous), surgical incisions and traumatic amputations.

The application of reduced or negative pressure creates a suction to remove excessive exudate from the wound, reducing the risk of maceration and infection. Furthermore, negative pressure stimulates the flow of fresh and oxygenated blood into the wound region as well as the formation of granulation tissue.

Currently, devices of this type use electrically powered pumps to obtain a suitable, constant negative pressure value. This implies an extremely high cost of the device itself. Furthermore, they cause a significant negative impact from an environmental point of view. In fact, medical devices for treating wounds by means of a suction process are disposable devices. For correct disposal, it is therefore necessary to separate the electronic components from the mechanical ones. Furthermore, devices of this type can be complicated to use, limiting the use thereof only to specialized personnel.

Devices that forego the use of an electric pump system are known in the literature.

For example, CN 202497590 U discloses a portable medical device for constant negative pressure drainage comprising a drainage container, a piston inserted into the container, and a constant-force mechanism formed by a spring structure. In order to manoeuvre the piston, an additional tool (a rear sliding bar) must be used to generate the negative pressure.

US 2019/0298899 A1 discloses a medical device for treating wounds capable of generating a substantially constant reduced pressure with a low tolerance for pressure fluctuations. The effectiveness of this instrument is, however, ensured by a complicated system of sensors and alarms to detect the emptying of the device in a timely manner.

US 2016/015592 A1 discloses an apparatus which applies micromechanical forces to a wound to accelerate the healing thereof. The apparatus comprises a pressure chamber with a sealing piston and a constant-force spring applied to the piston.

WO 2015/003194 A2 discloses a fluid drainage device comprising a volume for receiving the fluid with a conduit and a sealed piston with the conduit. The movement of the piston causes a change in volume for the fluid aspiration.

US 2016/354595 A1 discloses a valve system with a three-outlet connector to be connected to a system comprising a syringe for aspirating a fluid.

US 6,174,306 B1 discloses a device for sealing a wound covered by a film. The device comprises a drainage pipe coupled to a pump system and can be transportable.

US 2018/353660 A1 discloses a medical aspirator device having a cylindrical housing within which a piston and an elastic element slide to create a constant force to be used preferably in the operating theatre to maintain a constant aspiration.

US 2014/188061 A1 discloses a recharging negative-pressure wound therapy including a vacuum chamber, an ambient pressure chamber, a valve configured for unidirectional flow from the vacuum chamber to the ambient pressure chamber during a charging stroke, and a liquid filter configured to retain liquid in the pump.

Although the known art documents show the possibility of making negative pressure devices even without an electric pump, these devices are generally less effective since they are unlikely to reproduce a constant negative pressure value comparable to that of the electrical devices. Furthermore, in many cases they consist of a large number of additional movable components, making them complicated and subject to possible failure.

It is an object of the present invention to overcome in part or in whole the above-mentioned drawbacks of the known systems and to provide negative pressure devices which are effective, safe, economical and at the same time easy to use.

### DISCLOSURE OF THE INVENTION

A negative pressure device for the treatment of a wound is presented herein.

The device according to the present invention comprises a longitudinal housing having a first end configured to be connected to the wound and a second end opposite the first end. The longitudinal housing is a single-piece structure and is hollow and cylindrical.

The device further comprises a piston sliding inside the housing to generate the negative pressure and a constant-force mechanism configured to be loaded and to activate the piston. In particular, the constant-force mechanism is fixed at the second end of the longitudinal housing and to the sliding piston and is located inside the housing.

The device comprises a valve system for drawing air out of the housing when the piston slides to the first end of the housing and for bringing wound fluid into the housing when the piston slides to the second end of the housing.

In particular, the constant-force mechanism is entirely contained inside the longitudinal housing in which the piston slides. Furthermore, the second end of the longitudinal housing comprises a rear opening such as to allow the insertion of at least one finger of an individual's hand to manually slide the piston and load the constant-force mechanism.

Thereby, the device according to the present invention is capable of generating and controlling sub-atmospheric pressure inside the housing in the absence of an electrical power supply. By virtue of the opening placed at the second end of the housing, it is possible for example to use the finger of a hand to mechanically push the piston in a simple manner without having to resort to additional tools such as a special activation tool (rear bar). Furthermore, the use of the finger of one hand allows the user to control the suction process more easily and allows to have active sensation if the device becomes disconnected or if there were micro leaks. For example, the constant-force mechanism and thus the piston can be easily reloaded if necessary, simply with a finger.

In addition, since it is free of any electronic components, the device according to the present invention is considerably less expensive than the known art devices and easier to manufacture. Furthermore, during the disposal step, it is not necessary to separate components of different nature, but the device can be disposed of in its entirety among special medical waste.

It should be noted that the configuration according to which the constant-force mechanism is entirely contained inside the housing where the piston slides and the housing is a single piece improves the compactness and strength of the device. In fact, all the components necessary to generate the negative pressure (for example the piston and the constant-force mechanism), are entirely inserted in the housing which therefore also acts as a protective covering.

Although structurally simple to construct, the device according to the present invention is capable of achieving a constant negative pressure comparable to that achieved by devices employing electric pumps, for example reaching values between -50 mmHg and -150 mmHg.

The longitudinal housing can have several shapes. Preferably, the housing has a cylindrical shape with a circular section. Furthermore, the housing is hollow. This means that the housing comprises an internal central opening within which the piston slides and the constant-force mechanism is entirely housed. The first end of the housing is the one directed towards the wound and is connected by means of a tube or cannula system to a special wound dressing positioned in the patient's tissue. The dressing is composed of a filler, typically a three-dimensional hollow reticular foam structure, and a sealing layer to ensure airtight closure and maintain fluid communication between the wound and the housing of the device.

The piston is made of a gasket having a shape coinciding with the section of the housing so as to adhere to the inner walls of the housing as much as possible and a support fixed to the gasket which is in connection with the constant-force mechanism. Together with the inner walls and the first end of the housing, the gasket defines a pressure chamber inside which the negative pressure is generated. The movement of the piston varies the volume inside the housing, i.e., the pressure chamber. In particular, as the volume increases, the air pressure is reduced, resulting in a subsequent suction from the wound region.

To generate a constant negative pressure, the constant-force mechanism must be loaded so as to activate the piston and slide it inside the housing. By virtue of the rear opening positioned at the second end of the housing, i.e., the rear end of the device not directed towards the wound, the loading and activation easily occurs by the user (doctor, nurse, patient, etc.) inserting their finger through the opening and pushing the sliding piston directly. By pushing the piston, air exits from the housing, i.e., from the pressure chamber, through the valve system. Subsequently, the piston automatically flows backwards following the action of the constant-force mechanism, thereby expanding the volume inside the pressure chamber in the housing. This generates a suction at the wound and the wound fluid flows from the wound into the housing.

As already mentioned, the housing is a single piece (cylindrical and hollow) and the constant-force mechanism is completely placed inside the longitudinal housing. This greatly increases the compactness of the device. Such compactness allows a greater handling of the device by the user who can in principle treat the wound even using a single hand.

The rear opening can be of any size such that at least one finger of an adult person can be inserted therein. In an example, the rear opening can have a size which matches the cross-section of the housing. In the case of a cylindrical housing, the rear opening can be circular and can coincide with the cross section of the cylinder. Alternatively, the rear opening can be placed laterally, on the rear part of the housing, and have a roughly oval section. It should be noted that the rear opening is the only opening in the device which allows the insertion of one or more fingers to manually slide the piston.

In an example, the housing can comprise a curved rear cover at the second end which partially covers the back of the device. Thereby, the rear opening can be defined at least partially by the profile of the rear cover. Advantageously, the cover element can comprise a fixing element for fixing the constant-force mechanism to the housing.

According to an example, the constant-force mechanism can comprise at least one constant force spring fixed to the piston in a laminar manner. Preferably, in order to ensure better axial traction with the longitudinal housing, the constant-force mechanism comprises two constant-force springs.

According to a preferred example, the constant-force mechanism comprises a single constant-force spring mounted on a cylindrical support. The spring consists of a metallic band which can be wound around said cylindrical support, in which one end of the band is fixed to the cylindrical support and the other end of the band is provided with a connection element, in particular a through hole, to connect to the rear cover. Advantageously, the metal band rests on the curved profile of the rear cover when connected to said rear cover. In other words, the curvature of the rear curvature follows the stretched profile of the metallic band which can be wound around the cylindrical support, thus allowing the spring to generate the forces thereof all on the same axis, free of torque. This allows to use a single spring, as there is no rotational movement imprinted by the spring on the piston which could result in excessive grinding friction and a subsequent malfunction.

The piston comprises a main body with a first surface defining a pressure chamber with the first end of the housing. According to another example, the constant-force mechanism can be confined at least partially in an inner region of the main body of the piston. Thereby, the constant-force mechanism does not hinder the insertion of finger(s) from the rear opening to push the piston. For example, the cylindrical support to which one end of the constant-force mechanism (spring) is connected can be inserted in such an inner region of the main body of the piston.

In an example, the main body of the piston can comprise a second surface used to manually load the constant-force mechanism.

The valve system can be placed in different positions. For example, it may be located at one of the ends of the housing, at the piston itself, or at a side surface of the housing. The valve system can be for example insertable in a region of a wall of the housing. Furthermore, the valve system can be fixed or movable, for example integrally with the piston.

According to an example of the invention, the valve system can be positioned at the first end of the longitudinal housing.

Furthermore, the valve system can comprise one or more one-way valves. In the simplest configuration, the valve system can comprise a single one-way valve which allows air to vent out of the device, when the piston is pushed towards the first end.

According to an example, the valve system can include two one-way valves. In particular, a valve is activated when the piston is retracted and slides towards the second end of the housing. In this case, the pressure inside the pressure chamber in the housing is reduced, causing a flow from the outside (wound) towards the inside of the housing. The other valve is activated when the piston is pushed (for example by the user's finger) forwards, i.e., towards the first end of the housing. In this case, due to an increase in the pressure value, the air inside the pressure chamber in the housing exits to the outside. It should be noted that the valves of the valve system are mechanically activated without the use of electronic components.

According to an example, the valve system can comprise a cover element fixable to the first end of the longitudinal housing and a circular membrane positioned between the cover element and the first end of the longitudinal housing, i.e., the front of the housing itself. In particular, the cover element comprises a first opening for the entry of wound fluid and a second opening for the exit of air.

In order to ensure a more effective fluid exchange between the interior and exterior of the housing, the front portion of the longitudinal housing at the first end can comprise an inlet hole at the first opening of the cover element and an outlet hole at the second opening of the cover element. The circular membrane is configured to interpose between the first and second openings of the cover element and the inlet and outlet holes of the housing.

According to an example, the circular membrane can consist of a central disc element and a circumferential element concentric with the disc element and positioned externally thereto. The circumferential element acts as a gasket and is fixed between the cover element and the longitudinal housing. Thereby, the two one-way valves consist of two half-discs (or flaps) of the circular membrane, i.e., of the central disc element. Depending on the pressure generated inside the pressure chamber in the housing, i.e., depending on the movement of the piston, each half-disc can move in only one direction (upwards or downwards). The displacement of the half-discs causes the closing and opening of the two valves in an alternating manner. In other words, when one of the two valves is closed the other is open and vice versa. Specifically, the closing of the valves is due to the adhesion of the half-disc component of the circular membrane to the inlet or outlet hole on the first end of the housing.

In order to improve the adhesion of the membrane to the surface of the first end to close the corresponding holes, the front portion of the longitudinal housing at the first end can have a pointed profile and comprise two surfaces defining said tip. These surfaces are configured to be brought into alternate contact with one half of the circular membrane upon the sliding of the piston inside the longitudinal housing. The inlet hole of the housing is located on one of the two surfaces defining the tip while the outlet hole is located on the other surface defining the tip.

In order to create the system with two alternating valves, the front part of the housing can comprise an outer central edge and the cover element comprises an inner central edge. Thereby, the central disc of the circular membrane - which is positioned between the front of the housing and the cover element - can be divided into two half-discs following the crushing of said central disc between said outer central edge and said inner central edge.

In order to indicate the load state of the device as a function of the position of the piston, the device further comprises a scale indicator, preferably positioned along one side of the longitudinal housing.

These and other aspects of the present invention will become more apparent by reading the following description of some examples described below.
- Fig. 1a-b: show a front and rear schematic perspective depiction of the device according to an example of the invention.
- Fig. 2a-b: show a schematic perspective depiction of the device of figure 1.
- Fig. 3a-b: show a schematic perspective depiction of the piston and of the cover element of the device of figure 1.
- Fig. 4a-b: show a schematic depiction of the constant-force mechanism and the circular membrane of the device of figure 1.
- Fig. 5: shows a schematic depiction of the device of figure 1 with exploded view.
- Fig. 6a-b: show a schematic depiction of the back (a) and the front (b) of the device of figure 1.
- Fig. 7a-b: show a schematic depiction of the device of figure 1 in longitudinal section along the cut A-A of figure 6a.
- Fig. 8a-b: show a schematic depiction of the device of figure 1 in longitudinal section along the cut B-B of figure 6a.
- Fig. 9a-b: show a schematic longitudinal section depiction of the device of figure 1 (a) and a detail of the valve system (b).
- Fig. 10: shows a schematic depiction of the circular membrane.
- Fig. 11: shows a schematic longitudinal section view of the device of figure 1.
- Fig. 12a-b: show the front part of the housing with and without circular membrane.
- Fig. 13a-b: show the back part of the cover element with and without circular membrane.
- Fig. 14a-b: show a front and rear schematic perspective depiction of the device according to another example of the invention.
- Fig. 15a-b: show a schematic perspective depiction of the device of figure 14.
- Fig. 16a-b: show a schematic perspective depiction of the piston and of the cover element of the device of figure 14.
- Fig. 17a-b: show a schematic depiction of the constant-force mechanism and the circular membrane of the device of figure 14.
- Fig. 18: shows a schematic depiction of the device of figure 14 with exploded view.
- Fig. 19: shows a schematic depiction of the back of the device of figure 14.
- Fig. 20a-b: show a schematic depiction of the device of figure 14 in longitudinal section along the cut A-A of figure 19.
- Fig. 21a-b: show a schematic depiction of the device of figure 14 in longitudinal section along the cut B-B of figure 19.
- Fig. 22a-b: show a schematic longitudinal section depiction of the device of figure 14 (a) and a detail of the valve system (b).
- Fig. 23: shows a schematic depiction of the circular membrane.
- Fig. 24a-b: show a show a schematic longitudinal section depiction of the device of figure 14 with detail of the first end.
- Fig. 25a-b: show the front part of the housing with and without circular membrane.
- Fig. 26a-b: show the back part of the cover element with and without circular membrane.
- Fig. 27a-b: show a schematic depiction of the device of figure 14.

The main features of the device 1, 1' will be described in more detail in the following figures.

The device 1, 1' comprises a longitudinal housing 10. The housing 10 is cylindrical with a first end 101 configured to be connected to a wound and a second end 102. A piston 11 can slide inside the housing 10 which consists of a main body 21 and a first surface 22 defining a pressure chamber 17 with the first end 101 of the housing. The constant-force mechanism 12, 12' is connected to the piston 11 and to a point of the housing 10 at the second end 102 and can be loaded to activate the piston 11. In particular, the constant-force mechanism 12, 12' is inserted at least partially in an inner region 23 of the main body 21. It should be noted that a part of the constant-force mechanism 12, 12' is outside this inner region 23 and connects to a point of the housing at the second end 102. The main body 21 comprises a second surface 24 which is utilized to manually load the constant-force mechanism. In other words, through the rear opening 14 of the housing 10 it is possible to push (for example using one or more fingers of an individual's hand) the piston 11 by acting directly on the second surface 24 and thus load the constant-force mechanism 12, 12'. The main body 21 of the piston 11 comprises a first surface 22 which, together with the inner walls and the first end 101 of the housing 10, defines a pressure chamber 17 within which negative pressure is generated. The device 1 further comprises a valve system 13 which acts as a vent for the air when the piston 11 slides in the housing 10. The valve system 13 can be positioned in any region of the housing 10 (such as at one end, the piston, or a side wall) as long as it ensures the exit of air from the housing 10 when the piston slides towards the first end 101 and fluid entry in the housing 10 (i.e., inside the pressure chamber 17) when the piston slides towards the second end 102. More details on the operation of the device will be described with reference to the following examples.

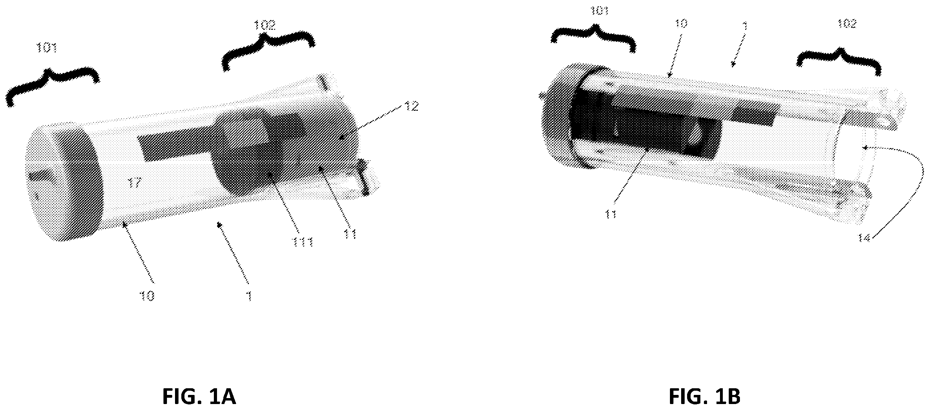

Figures 1a and 1b depict the negative pressure device 1 according to an example of the invention. The device 1 comprises a longitudinal housing 10 having a first end 101 configured to be connected to the wound and a second end 102 opposite the first end 101. The second end 102 of the longitudinal housing 10 comprises a rear opening 14 such as to allow the insertion of at least one finger of an individual's hand to manually slide a piston 11 inside the housing 10. The piston 11 (or rather the gasket 111 thereof) defines together with the inner walls and the first end 101 of the housing 10 a pressure chamber 17 inside which the negative pressure is generated. The movement of the piston 11 varies the volume inside the housing 10, i.e., the pressure chamber 17. In particular, as the volume increases, the air pressure is reduced, resulting in a subsequent suction from the wound region. Figure 1a shows in particular the situation where the piston 11 is at the second end 102 of the housing 10 and the volume of the pressure chamber 17 is maximum. In this case, the constant-force mechanism 12 is not loaded. Figure 1b instead shows the situation where the piston 11 is at the first end 101 of the housing 10 and the volume of the pressure chamber 17 is minimal. The sliding of the piston 11 towards the first end 101 of the housing is achieved by a thrust thereof through the rear opening 14. Thereby, the constant-force mechanism 12, which is fixed to the second end 102 of the housing 10 and at the same time attached to the piston 11, is loaded and the device 1 is ready for the suction process.

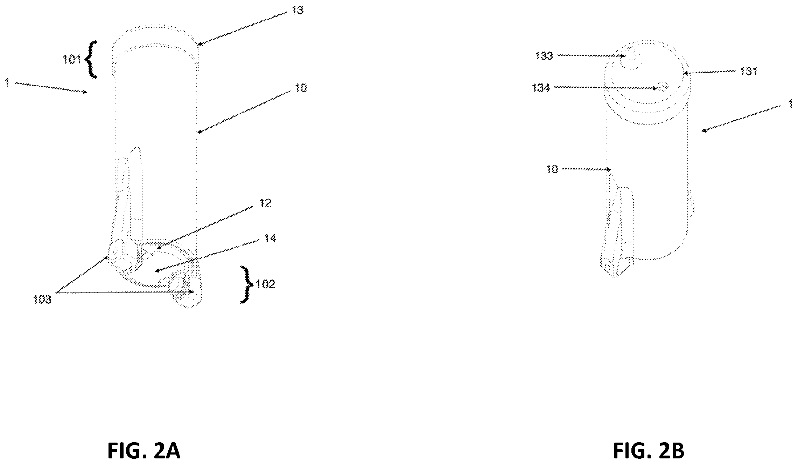

Figures 2a and 2b show a perspective view of the device of figure 1. The housing 10 and thus the pressure chamber 17 comprises two fixing points 103 at the second end 102 to allow the constant-force mechanism 12 to be fixed with the housing 10. Furthermore, the device 1 comprises a valve system 13 at the first end 101. In particular, the valve system 13 comprises a cover element 131 having a first opening 133 connected by means of a tube or cannula system to a special wound dressing positioned in the patient's tissue and a second opening 134 for the exit of air from the pressure chamber 17.

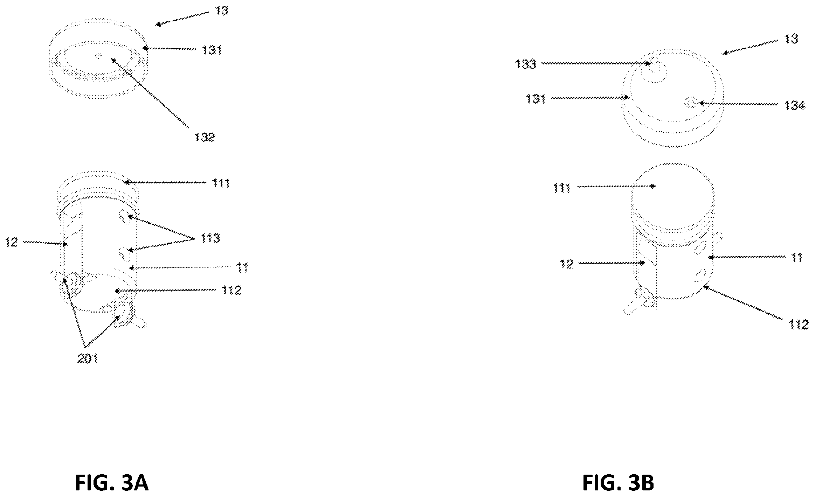

Figures 3a and 3b show a perspective view of the piston 11 and valve system 13. The piston 11 consists of a gasket 111 and a support 112. The constant-force mechanism 12 is connected to the support 112 of the piston 11 by means of first connectors 113 (described in detail in the following figures) and is connected to the housing 10 by means of second connectors 201 which interact with the fixing points 103 of the housing 10. The valve system 13 is composed of a cover element 131 and a circular membrane 132.

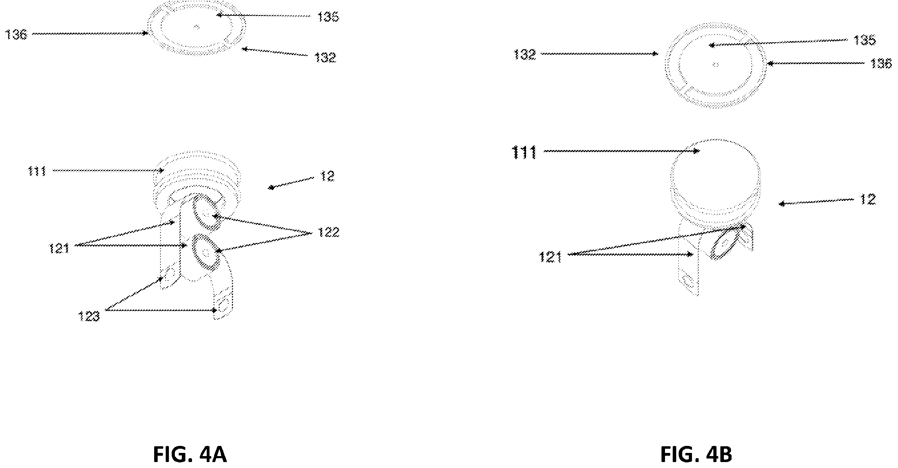

Figures 4a and 4b show a perspective view of the constant-force mechanism 12 and the circular membrane 132. The constant-force mechanism 12 according to an example comprises two constant-force springs 121 arranged one above the other in the longitudinal direction of the housing 10 and each mounted on a cylindrical support 122. Specifically, each constant-force spring 121 is made of a metallic band wound around said cylindrical support 122, in which one end of the band is fixed to the cylindrical support 122 while the other end is provided with a connecting element 123, for example a through hole, to connect to the second connectors 201 of the constant-force mechanism 12. The two springs 121 are wound around the supports 122 according to two opposite winding directions. The circular membrane 132 consists of a central disc element 135 and a circumferential element 136 concentric with the disc element 135 and positioned externally thereto. More details of the circular membrane 132 will be described in the following figures.

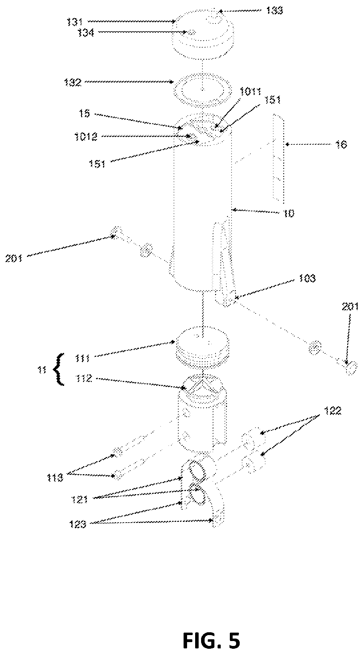

Figure 5 shows an exploded view of the device 1 according to the example of figures 1-4. As shown above, the cover element 131 comprises a first opening 133 connected by means of a tube or cannula system to the patient's wound and a second opening 134 for the exit of air from inside the pressure chamber 17. Similarly, the housing 10 comprises an inlet hole 1011 that can be coupled to the first opening 133 and an outlet hole 1012 that can be coupled to the second opening 134. According to this example, the housing 10 has a cylindrical shape and therefore the gasket 111 of the piston 11 has a circular shape of the same size as the cross-section of the housing 10 so as to perfectly adhere to the inner walls of the housing 10. From the figure it can be noted that the first connectors 113 between the piston 11, i.e., the support 112, and the constant-force mechanism 12, i.e., the springs 121, consist of two pins which pass through two holes in the support 112 and are fixed to the cylindrical supports 122. Furthermore, from the figure it can be noted that the second connectors 201 between the housing 10 at the second end 102 and the constant-force mechanism 12, i.e., the springs 121, consist of two pins which pass through two holes 103 in the housing 10 and connect to the connecting elements 123 of the springs 121 at one of the ends thereof. The scaled indicator 16 extending longitudinally outside the housing 10 marks the load state of the device.

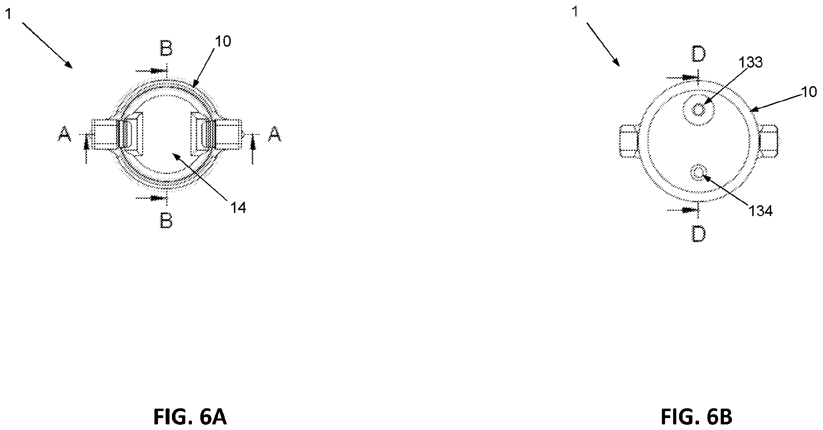

Figure 6a shows the back of the device 1. Specifically, the figure shows the presence of a rear opening 14 through which one or more fingers of a hand can be inserted to activate and load the constant-force mechanism 12. Figure 6b instead shows the front part of the device 1 in which the first and second openings 133, 134 are present for the connection with the wound and for the exit of air from the pressure chamber 17.

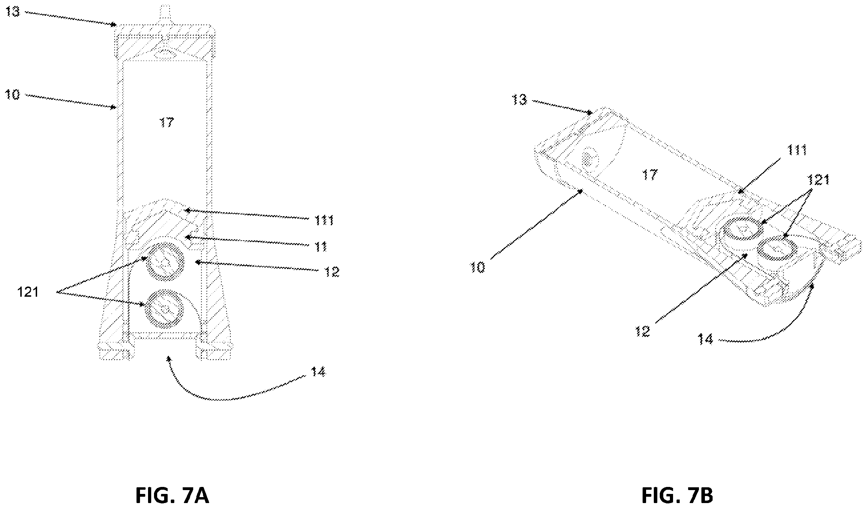

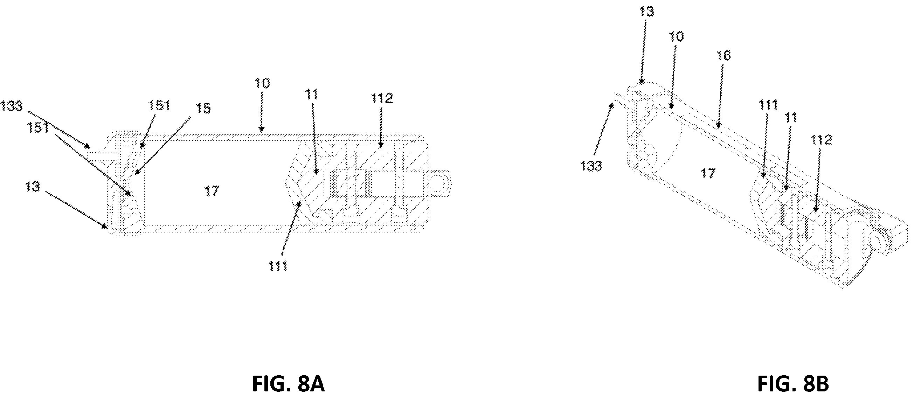

Figures 7a and 7b show the device 1 according to a longitudinal section along the cut A-A of figure 6a, while figures 8a and 8b illustrate the device 1 according to a longitudinal section along the cut B-B of figure 6a. These figures show the details of the interior of the device 1 and specifically, the pressure chamber 17 represented by the volume defined by the piston 11, i.e., the gasket 111, the inner walls of the housing 10 and the first end 101 of the housing 10, i.e., the upper part of the housing 10. Furthermore, the figures show the arrangement of the springs 121 forming the constant-force mechanism 12 and the connection thereof with the piston 11, i.e., the support 112.

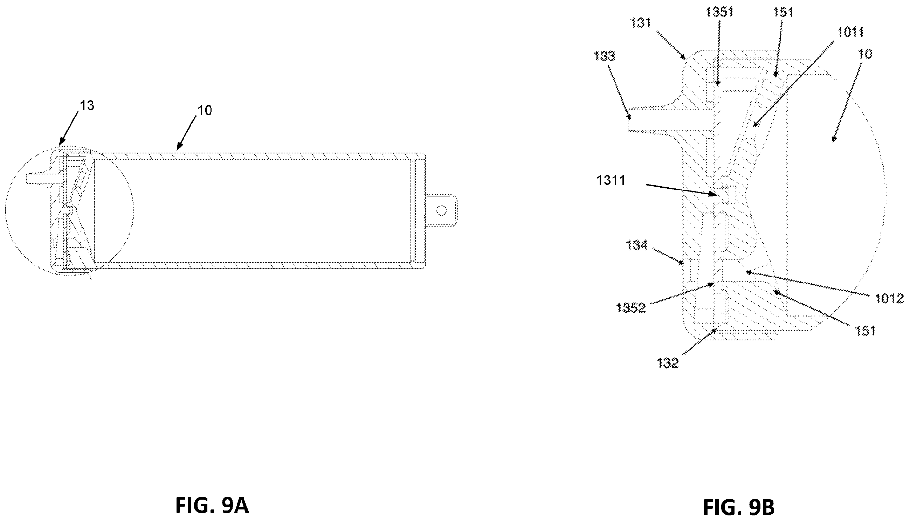

Figures 9a and 9b show the device 1 in longitudinal section and a detail of the valve system 13 positioned at the first end 101 of the housing 10. From figure 9b it can be noted that the housing 10 comprises an inlet hole 1011 and an outlet hole 1012 connected to the valve system 13. The circular membrane 132 is configured to fit the surfaces of the cover element 131 and the top of the housing 10. In particular, the circular membrane comprises two half-discs 1351 and 1352 which can move alternately to close or open the air or fluid passages determined by the first and second openings 133, 134 on the cover element 131 and by the inlet and outlet hole 1011, 1012 on the upper part of the housing 10.

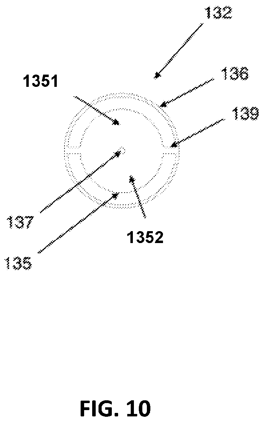

Figure 10 shows the circular membrane 132 in detail. The membrane 132 consists of a central disc element 135 and a circumferential element 136 concentric with the disc element 135 and positioned externally thereto. The circumferential element 136 acts as a gasket and is fixed between the cover element 131 and the longitudinal housing 10, i.e., the top of the housing 10. The membrane 132 comprises a central hole 137 for improving the alignment thereof at the cover element 131 and the housing 10 and comprises at least two side edges 139 connecting the central disc 135 to the circumference element 136. The central disc 135 is divided into two half-discs 1351, 1352 which may be referred to as "semi-valves". The division of the central disc 135 in two is ensured by the crushing of said central disc 135 between an outer central edge 153 present on the upper part of the housing 10, i.e., on the pointed profile 15 defined by the inclined surfaces 151 and an inner central edge 138 present on the cover element 131.

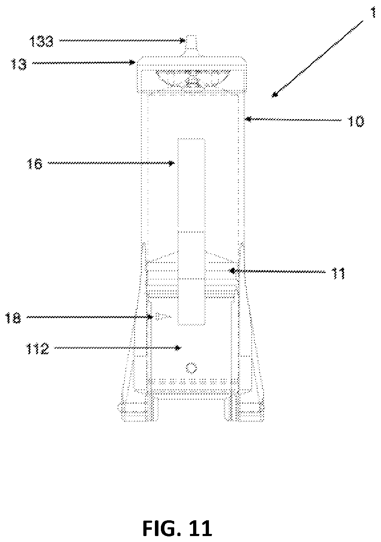

Figure 11 shows a schematic depiction of the device 1 in which the presence of a scaled indicator 16 positioned on the surface of the housing 10 is highlighted, for example the outer surface and a corresponding piston indicator 18 placed on the support 112 of the piston 11. The position of the latter indicator 18 changes depending on the load state of the device 1, i.e., depending on the position of the piston 11 relative to the first end 101 or second end 102 of the housing 10. Advantageously, the scaled indicator 16 defines the load state of the device 10 with different colours.

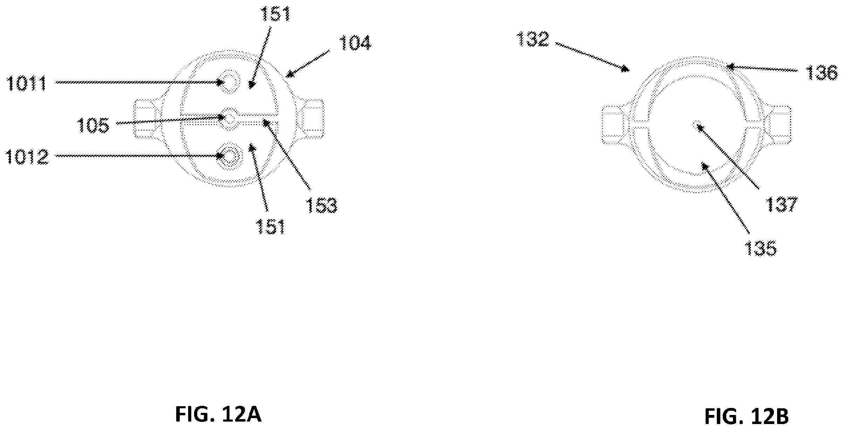

Figure 12a shows the front portion 104 of the housing 10 which is defined by a pointed profile 15 with two inclined surfaces 151 (consider, for example, the sections in figure 8a). The inlet hole 1011 and the outlet hole 1012 are on these surfaces. The tip of the front portion 104 of the housing 10 is defined by the centre edge 153 used to crush the circular membrane 132 and divide it into two half-discs 1351, 1352. The front portion 104 of the housing 10 further comprises a central hole 105 for aligning with the membrane 132 by means of the corresponding hole 137.

Figure 12b shows the front 104 of the housing 10 of figure 12a on which the membrane 132 lies. The circumferential element 136 of the membrane 132 acts as a gasket while the central disc 135 acts as an alternating valve as explained above.

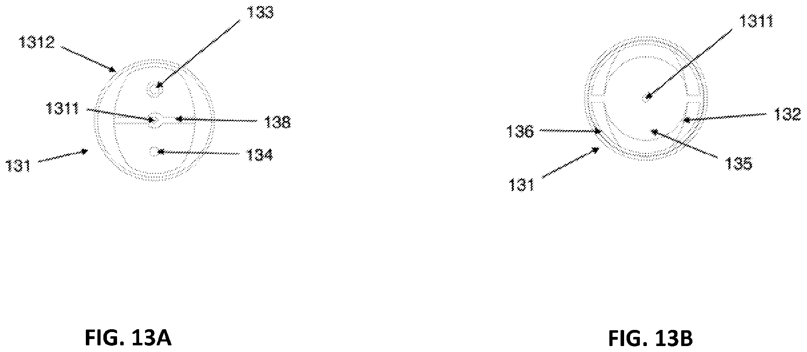

Figure 13a shows the back of the cover element 131 having the first opening 133 and the second opening 134. In particular, the cover element comprises an inner pin 1311 which serves for the alignment of the membrane 132 with the front portion 104 of the housing 10 by inserting said pin 1311 with the central holes 137 and 105, respectively of the membrane 132 and the front portion 104 of the housing 10. Furthermore, there is a central inner edge 138 which serves to define the two half-discs 1351 and 1352 of the circular membrane 132 following the crushing of the central disc 135 of the membrane 132 between said central inner edge 138 and the central outer edge 153 of the front 104 of the housing 10. To ensure the maximum seal, the outer part of the diameter 1312 of the cover element 131 is coupled with the circumferential element 136 of the circular membrane 132.

Figure 13b shows the back of the cover element 131 of figure 13a on which the membrane 132 lies. The circumferential element 136 of the membrane 132 acts as a gasket while the central disc 135 acts as an alternating valve as explained above. The inner pin 1311 passes through the hole 137 of the membrane 132.

It should be noted that the device 1 of figures 1-13 shows a valve system 13 placed at the first end 101. However, as described above, the valve system 13 can advantageously be positioned differently in other parts of the device 1.

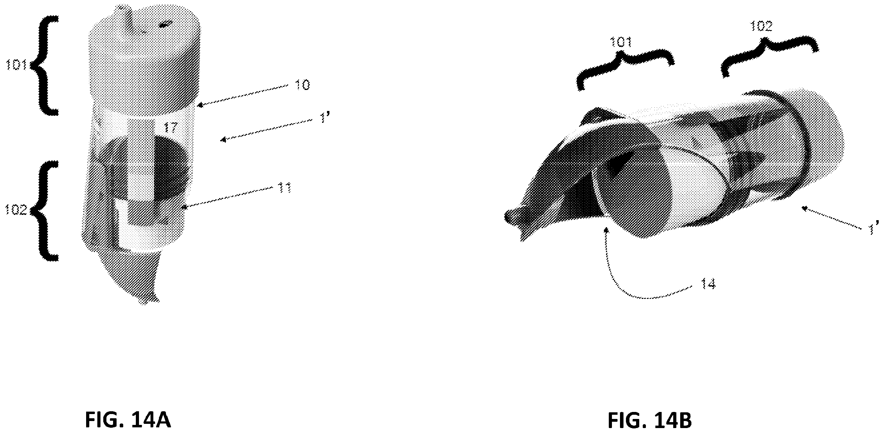

Figures 14a and 14b depict the negative pressure device 1' according to another example of the invention. This device differs from the previous one essentially by the mere fact that the constant-force mechanism consists of a single spring. Thus, identical components between the two devices will not necessarily be described again. The reference numbers of identical components therefore remain unchanged.

As in the case of the device 1, of figures 1-13, the device 1' comprises a longitudinal housing 10 having a first end 101 configured to be connected to the wound and a second end 102 opposite the first end 101. The second end 102 of the longitudinal housing 10 comprises a rear opening 14 such as to allow the insertion of at least one finger of an individual's hand to manually slide a piston 11 inside the housing 10. The piston 11 (or rather the gasket 111 thereof) defines together with the inner walls and the first end 101 of the housing 10 a pressure chamber 17 inside which the negative pressure is generated. The movement of the piston 11 varies the volume inside the housing 10, i.e., the pressure chamber 17. In particular, as the volume increases, the air pressure is reduced, resulting in a subsequent suction from the wound region.

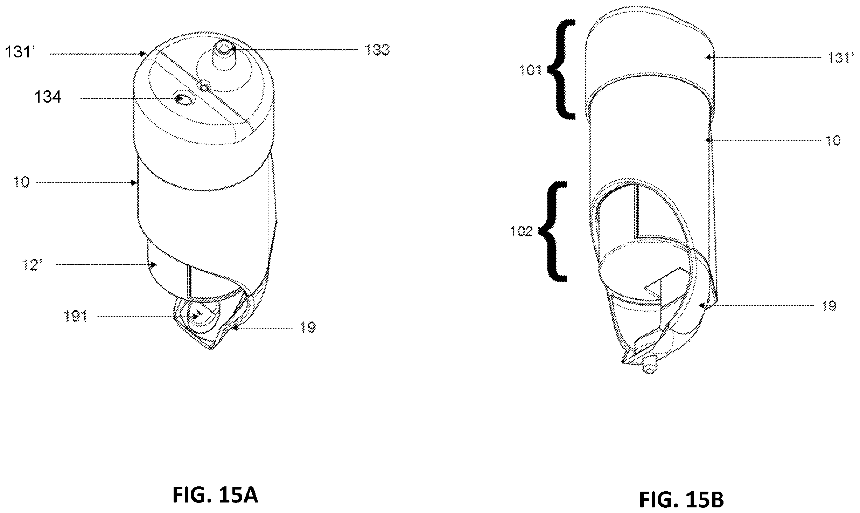

Figures 15a and 15b show a perspective view of the device of figure 14. The housing 10 and therefore the pressure chamber 17 comprises a curved rear cover 19 partially covering the back of the device 1' at the second end 102. There is a fixing element 191 for the constant-force mechanism 12' on this cover 19. The cover element 131' instead covers the first end 101 of the device 1'.

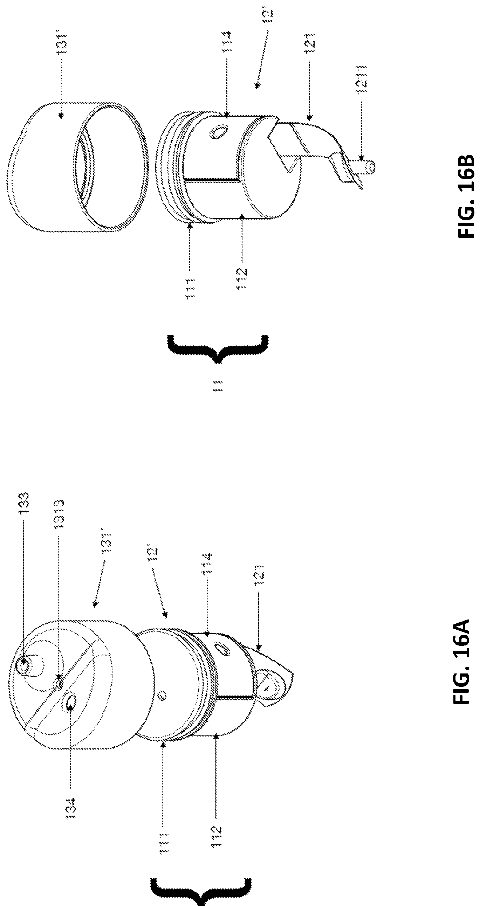

Figures 16a and 16b show a perspective view of the piston 11 and the cover element 131'. The piston 11 consists of a gasket 111, a support 112 and a component 114. From the figure it can be noted that the constant-force mechanism 12' comprises a single spring 121 which can be fixed at an end thereof to the rear cover 19 by corresponding fixing means 1211. In addition to the first and second openings 133, 134, the cover element 131' comprises a central alignment hole 1313.

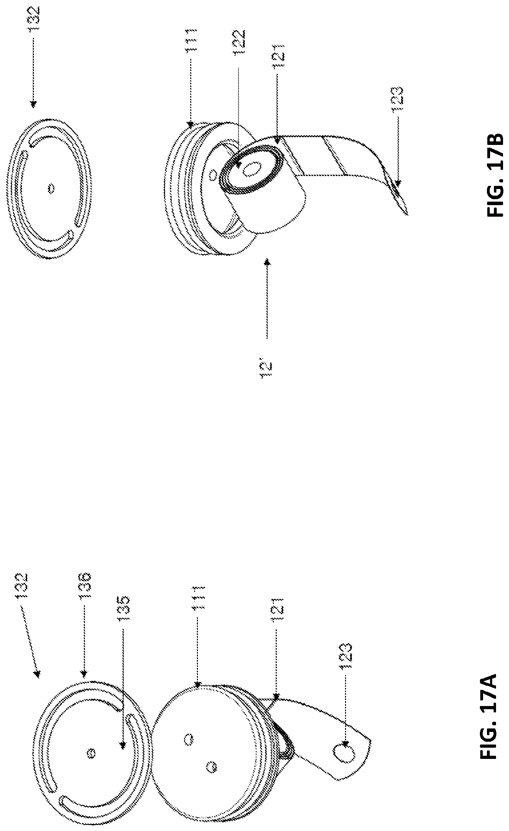

Figures 17a and 17b show a perspective view of the constant-force mechanism 12' and the circular membrane 132. The constant-force mechanism 12' according to this example comprises a constant-force spring 121 mounted on a cylindrical support 122. Specifically, the constant force spring 121 is made of a metallic band wound around said cylindrical support 122, in which one end of the band is fixed to the cylindrical support 122 while the other end is provided with a connecting element 123, for example a through hole, to connect to the rear cover 19 by fixing means 1211. The circular membrane 132 consists of a central disc element 135 and a circumferential element 136 concentric with the disc element 135 and positioned externally thereto.

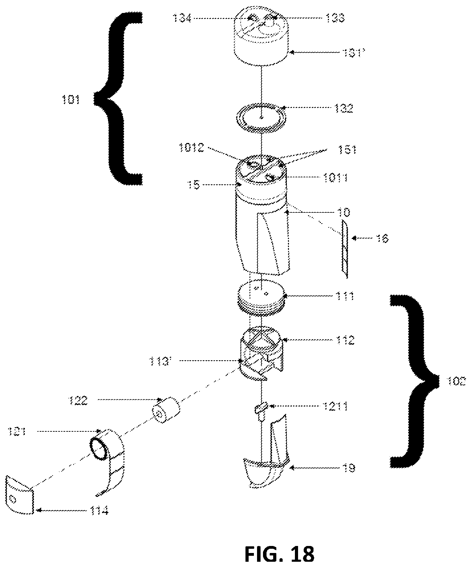

Figure 18 shows an exploded view of the device 1 according to the example of figures 14-17. As shown above, the cover element 131' comprises a first opening 133 connected by means of a tube or cannula system to the patient's wound and a second opening 134 for the exit of air from inside the pressure chamber 17. Similarly, the housing 10 comprises an inlet hole 1011 that can be coupled to the first opening 133 and an outlet hole 1012 that can be coupled to the second opening 134. According to this example, the housing 10 has a cylindrical shape and therefore the gasket 111 of the piston 11 has a circular shape of the same size as the cross-section of the housing 10 so as to perfectly adhere to the inner walls of the housing 10. From the figure it can be noted that the device 1' comprises a pin 113' which extends from the support 112, passes through the cylindrical support 122 and is fixed to the additional component 114 which holds everything in position. The figure also shows the presence of a scaled indicator 16 positioned outside on the housing 10 to indicate the load state of the device 1'.

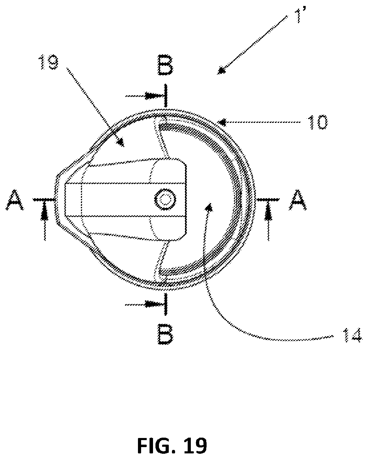

Figure 19 shows the back of the device 1'. Specifically, the figure shows the presence of a rear opening 14 through which one or more fingers of a hand can be inserted to activate the constant-force mechanism 12'. From the figure it can be seen that the rear cover 19 partially covers the opening 14.

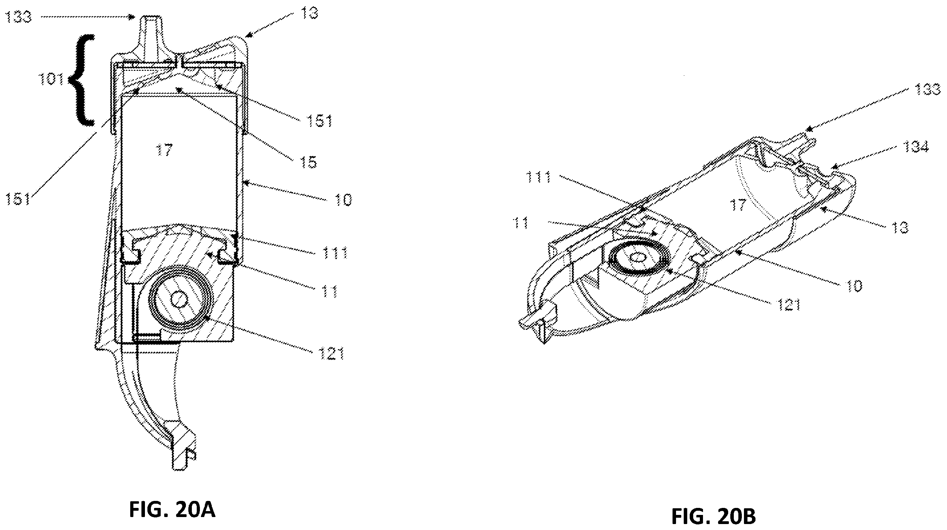

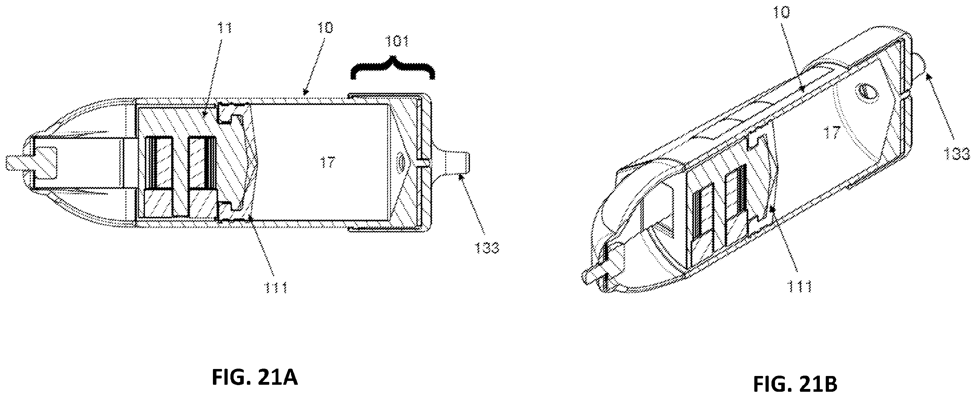

Figures 20a and 20b show the device 1 according to a longitudinal section along the cut A-A of figure 19, while figures 21a and 21b illustrate the device 1 according to a longitudinal section along the cut B-B of figure 19. These figures show the details of the interior of the device 1' and specifically, the pressure chamber 17 represented by the volume defined by the piston 11, i.e., the gasket 111, the inner walls of the housing 10 and the first end 101 of the housing 10, i.e., the upper part of the housing 10. Furthermore, the figures show the arrangement of the spring 121 forming the constant-force mechanism 12 and the connection thereof with the piston 11, i.e., the support 112.

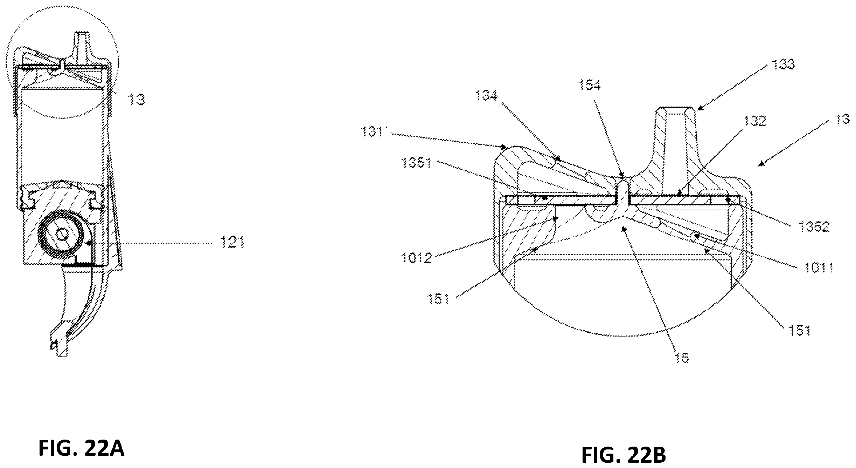

Figures 22a and 22b show the device 1' in longitudinal section and a detail of the valve system 13 positioned at the first end 101 of the housing 10. From figure 22b it can be noted that the housing 10 comprises an inlet hole 1011 and an outlet hole 1012 connected to the valve system 13. The circular membrane 132 is configured to fit the surfaces of the cover element 131' and the top of the housing 10. In particular, the circular membrane 132 comprises two half-discs 1351 and 1352 which can move alternately to close or open the air or fluid passages determined by the first and second openings 133, 134 on the cover element 131' and by the inlet and outlet hole 1011, 1012 on the upper part of the housing 10. To hold in position the membrane 132, the cover element 131' and the housing 10 in place, the upper part of the housing 10 comprises a pin 154 which passes through the central hole 137 of the membrane 132 and the central hole 1313 of the cover element 131'. It can also be noted from the figure that the profile of the outer surface of the cover element 131' is not flat but comprises a raised region at the second opening 134 to facilitate the exit of air. This configuration of the cover element 131' can also be applied to the example of figures 1-13 described above. In particular, the circumferential element 136 is configured so that it can be crushed between the covering element 131' and the housing 10.

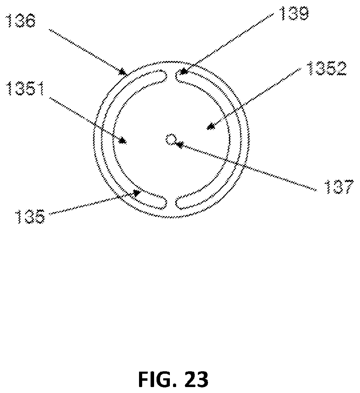

Figure 23 shows the circular membrane 132 in detail. As in the previous example, the membrane 132 consists of a central disc element 135 and a circumferential element 136 concentric with the disc element 135 and positioned externally thereto. The circumferential element 136 acts as a gasket and is fixed between the cover element 131 and the longitudinal housing 10, i.e., the top of the housing 10. The membrane 132 comprises a central hole 137 for improving the alignment thereof at the cover element 131' and the housing 10 and comprises at least two side edges 139 connecting the central disc 135 to the circumference element 136. The central disc 135 is divided into two half-discs 1351, 1352 which may be referred to as "semi-valves". The division of the central disc 135 in two is ensured by the crushing of said central disc 135 between a central edge 153 present on the upper part of the housing 10, i.e., on the pointed profile 15 defined by the inclined surfaces 151 and an inner central edge 138 present on the cover element 131.

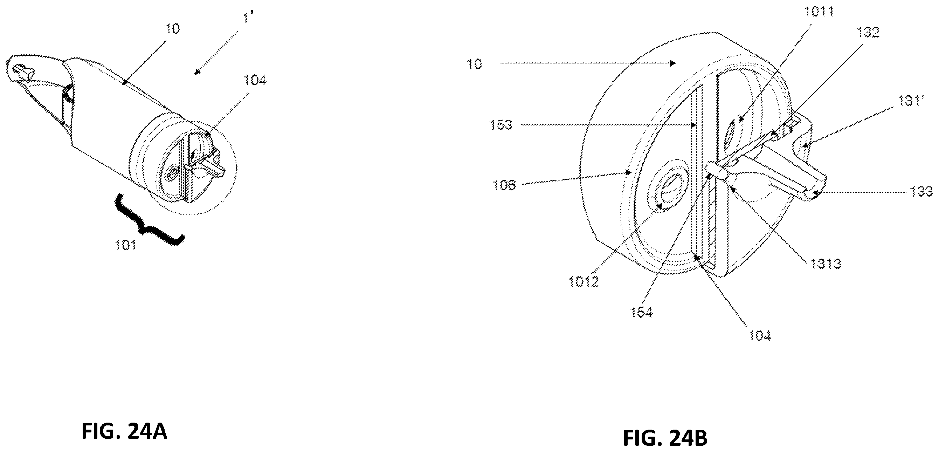

Figures 24a and 24b show a schematic depiction of the device 1' with detail on the front part 104 of the housing 10 cut to 3/4. Figure 24b shows the alignment of the circular membrane 132 with respect to the housing 10 and the cover element 131' determined by inserting the pin 154 in the central holes 137 and 1313 of the central membrane 132 and cover element 131', respectively.

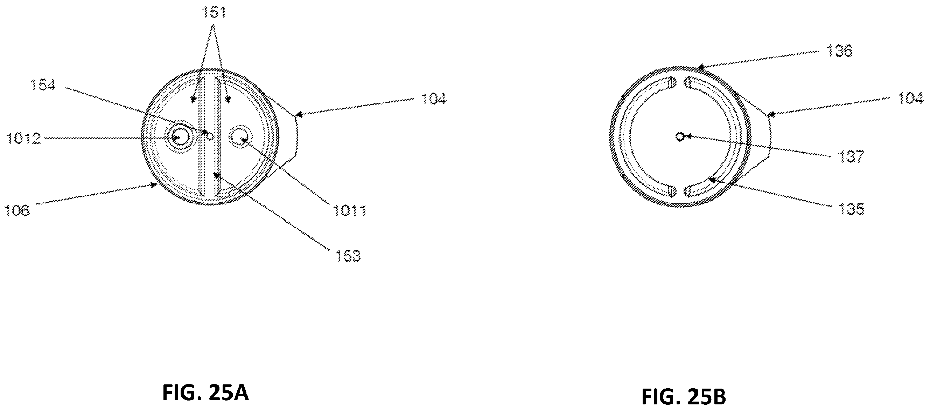

Figure 25a shows the front 104 of the housing 10 which is defined by a pointed profile 15 with two inclined surfaces 151. The inlet hole 1011 and the outlet hole 1012 are present on these surfaces 151 (consider for example the section in Figure 20a). The tip of the front portion 104 of the housing 10 is defined by the centre edge 153 used to crush the circular membrane 132 and divide it into two half-discs 1351, 1352. The front portion 104 of the housing 10 further comprises a central pin 154 for aligning with the membrane 132 by means of the corresponding hole 137.

Figure 25b shows the front 104 of the housing 10 of figure 25a on which the membrane 132 lies. In particular, the membrane 132 lies on the outer portion 106 of the housing 10. The circumferential element 136 of the membrane 132 acts as a gasket while the central disc 135 acts as an alternating valve as explained above.

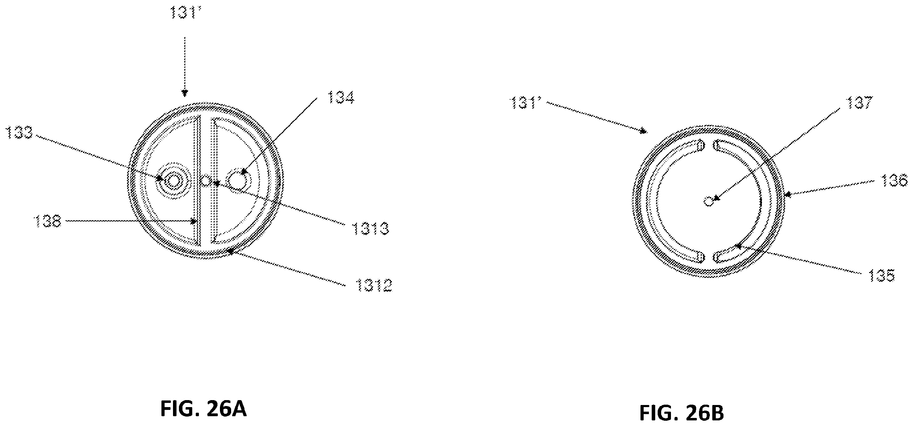

Figure 26a shows the back of the cover element 131' having the first opening 133 and the second opening 134. In particular, the cover element comprises a central hole 1313 which serves for aligning the membrane 132 with the front part 104 of the housing 10 by inserting the pin 154 of the front part 104 of the housing 10. Furthermore, there is a central inner edge 138 which serves to define the two half-discs 1351 and 1352 of the circular membrane 132 following the crushing of the central disc 135 of the membrane 132 between said central inner edge 138 and the central edge 153 of the front 104 of the housing 10. To ensure the maximum seal, the outer part of the diameter 1312 of the cover element 131' is coupled with the circumferential element 136 of the circular membrane 132.

Figure 26b shows the back of the cover element 131' of figure 13a on which the membrane 132 lies. The circumferential element 136 of the membrane 132 acts as a gasket while the central disc 135 acts as an alternating valve as explained above. The inner pin 154 passes through the hole 137 of the membrane 132.

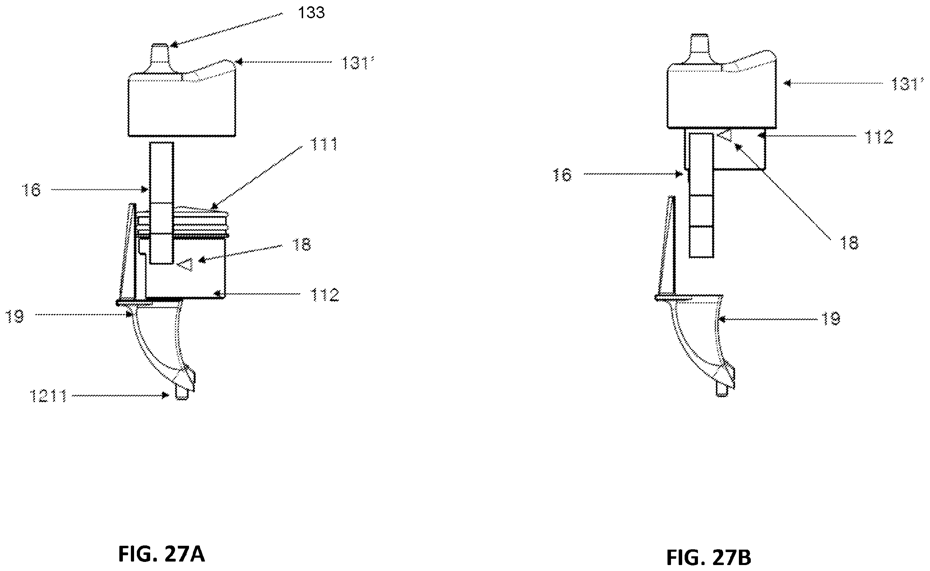

Figures 27a 27b show a schematic depiction of the device 1' in which the presence of a scaled indicator 16 positionable on the surface of the housing 10 is highlighted, for example the outer surface and a corresponding piston indicator 18 placed on the support 112 of the piston 11. It should be noted that these figures show only some components of the device 1'. For example, the housing 10 and the constant-force mechanism 12' are omitted from the figures. The position of the indicator 18 changes depending on the load state of the device 1, i.e., depending on the position of the piston 11 relative to the first end 101 or second end 102 of the housing 10. Advantageously, the scaled indicator 16 defines the load state of the device 10 with different colours.

It should be noted that the device 1' of figures 14-27 shows a valve system 13 placed at the first end 101. However, as described above, the valve system 13 can advantageously be positioned differently in other parts of the device 1.

A person skilled in the art can perform several and further modifications and variants to the device 1, 1' described above, in order to satisfy further and contingent needs, all said modifications and variants however included within the scope of protection of the present invention as defined by the appended claims.

## Claims

1. Negative pressure device (1, 1') for treating a wound and removing fluid from said wound, the device (1, 1') comprising:
a longitudinal housing (10) having a first end (101) configured to be connected to the wound and a second end (102) opposite the first end (101), wherein the longitudinal housing (10) is a structure in a single piece, hollow and cylindrical,
a piston (11) configured to slide inside the housing (10) to generate the negative pressure,
a constant-force mechanism (12, 12') configured to be loaded and operated by the piston (11), wherein said constant-force mechanism (12, 12') is fixed at the second end (102) of the longitudinal housing (10) and to the sliding piston (11) and is arranged inside the housing (10), and
a valve system (13) for drawing air out of the housing (10) when the piston (11) slides towards the first end (101) of the housing (10) and for bringing wound fluid into the housing (10) when the piston (11) slides towards the second end (102) of the housing (10),
the constant-force mechanism (12, 12') is entirely contained inside the longitudinal housing (10) where the piston (11) slides and
**characterized in that**
the second end (102) of the longitudinal housing (10) comprises a rear opening (14) having a size such that one or more fingers of an individual's hand are insertable therein to manually slide the piston (11) and load the constant-force mechanism (12, 12').

2. Device (1') according to claim 1, wherein the housing (10) comprises a rear cover (19) curved at the second end (102) partially covering the back of the device (1').

3. Device (1') according to claim 2, wherein the cover element (19) comprises a fixing element (191) for fixing the constant-force mechanism (12') to the housing (10).

4. Device (1, 1') according to one of the preceding claims, wherein the constant-force mechanism (12, 12') comprises at least one constant-force spring (121) fixed to the piston (11) in a laminar manner which can be activated by the pressure exerted by one or more fingers of a single user.

5. Device (1') according to claims 2, 3 and 4, wherein the constant-force mechanism (12') comprises a single constant-force spring mounted on a cylindrical support (122), wherein the spring is formed by a metallic band which can be wound around said cylindrical support (122), in which one end of the band is fixed to the cylindrical support (122) and the other end of the band is provided with a connecting element (123), in particular a through hole, for connecting to the rear cover (19), wherein the metallic band follows the curved profile of the rear cover (19) when connected to said rear cover (19).

6. Device (1, 1') according to one of the preceding claims, wherein the piston (11) comprises a main body (21) with a first surface (22) defining a pressure chamber (17) with the first end (101) of the housing (10) and the constant-force mechanism (12, 12') is confined, at least partially, in an inner region (23) of said main body (21) of the piston (11).

7. Device (1, 1') according to claim 6, wherein the main body (21) of the piston (11) comprises a second surface (24) used to manually load the constant-force mechanism (12, 12').

8. Device (1, 1') according to one of the preceding claims, wherein the valve system (13) is placed at the first end (101) of the longitudinal housing (10).

9. Device (1, 1') according to one of the preceding claims, wherein the valve system (13) comprises two one-way valves.

10. Device (1, 1') according to one of the preceding claims, wherein the valve system (13) comprises a cover element (131, 131') fixable to the first end (101) of the longitudinal housing (10) and a circular membrane (132) positioned between the cover element (131, 131') and the first end (101) of the longitudinal housing (10).

11. Device (1, 1') according to claim 10, wherein the cover element (131, 131') comprises a first opening (133) for the entry of wound fluid and a second opening (134) for the exit of air.

12. Device (1, 1') according to claim 11, wherein the longitudinal housing (10) comprises a front portion (104) at the first end (101), wherein said front portion (104) comprises an inlet hole (1011) at the first opening (133) of the cover element (131, 131') and an outlet hole (1012) at the second opening (134) of the cover element (131, 131').

13. Device (1, 1') according to one of claims 10-12, wherein the circular membrane (132) consists of a central disc element (135) and a circumferential element (136) concentric with the disc element (135) and positioned externally thereto, wherein the circumferential element (136) acts as a gasket and is fixed between the cover element (131) and the longitudinal housing (10).

14. Device (1, 1') according to one of claims 10-13, wherein the longitudinal housing (10) comprises a front part (104) at the first end (101), wherein said front part (104) has a pointed profile (15) and comprises two surfaces (151) defining a tip and which are configured to be alternately contacted with a half of the circular membrane (132) following the sliding of the piston (11) inside the longitudinal housing (10).

15. Device (1, 1') according to one of claims 13-14, wherein the front portion (104) of the housing comprises an outer central edge (153), the cover element (131, 131') comprises an inner central edge (138) and the central disc (135) of the circular membrane (132) is divided into two half-discs (1351, 1352) following the crushing of said central disc (135) between said outer central edge (153) and said inner central edge (138).

## Patentansprüche

1. Unterdruckvorrichtung (1, 1') zum Behandeln einer Wunde und Entfernen von Flüssigkeit aus der Wunde, wobei die Vorrichtung (1, 1') umfasst:
ein Längsgehäuse (10) mit einem ersten Ende (101), das so konfiguriert ist, dass es mit der Wunde verbunden werden kann, und einem zweiten Ende (102) entgegengesetzt zu dem ersten Ende (101), wobei das Längsgehäuse (10) eine Struktur in einem einzigen Stück, hohl und zylindrisch, ist,
einen Kolben (11), der so konfiguriert ist, dass er innerhalb des Gehäuses (10) gleiten kann, um den Unterdruck zu erzeugen,
einen Konstantkraft-Mechanismus (12, 12'), der so konfiguriert ist, dass er durch den Kolben (11) geladen und betätigt wird, wobei der Konstantkraft-Mechanismus (12, 12') an dem zweiten Ende (102) des Längsgehäuses (10) und an dem Gleitkolben (11) befestigt ist und im Inneren des Gehäuses (10) angeordnet ist, und
ein Ventilsystem (13) zum Absaugen von Luft aus dem Gehäuse (10), wenn der Kolben (11) zum ersten Ende (101) des Gehäuses (10) hin gleitet, und zum Befördern von Wundflüssigkeit in das Gehäuse (10), wenn der Kolben (11) zum zweiten Ende (102) des Gehäuses (10) hin gleitet,
wobei der Konstantkraft-Mechanismus (12, 12') vollständig in dem Längsgehäuse (10), in dem der Kolben (11) gleitet, aufgenommen ist, und
**dadurch gekennzeichnet, dass**
das zweite Ende (102) des Längsgehäuse (10) eine hintere Öffnung (14) mit einer Größe umfasst, sodass ein oder mehrere Finger der Hand eines Individuums darin einsteckbar sind, um den Kolben (11) manuell zu verschieben und den Konstantkraft-Mechanismus (12, 12') zu laden.

2. Vorrichtung (1') gemäß Anspruch 1, wobei das Gehäuse (10) eine am zweiten Ende (102) gekrümmte hintere Abdeckung (19) umfasst, welche die Rückseite der Vorrichtung (1') teilweise abdeckt.

3. Vorrichtung (1') gemäß Anspruch 2, wobei das Abdeckelement (19) ein Befestigungselement (191) zum Befestigen des Konstantkraft-Mechanismus (12') an dem Gehäuse (10) umfasst.

4. Vorrichtung (1, 1') gemäß einem der vorhergehenden Ansprüche, wobei der Konstantkraft-Mechanismus (12, 12') mindestens eine laminar am Kolben (11) befestigte Konstantkraftfeder (121) umfasst, die durch den Druck aktiviert werden kann, der durch ein oder mehrere Finger eines einzelnen Anwenders ausgeübt wird.

5. Vorrichtung (1') gemäß Ansprüchen 2, 3 und 4, wobei der Konstantkraft-Mechanismus (12') eine einzelne Konstantkraftfeder, die auf einer zylindrischen Halterung (122) montiert ist, umfasst, wobei die Feder aus einem Metallband besteht, das um die zylindrische Halterung (122) gewunden werden kann, indem ein Ende des Bandes an der zylindrischen Halterung (122) befestigt wird und das andere Ende des Bandes mit einem Verbindungselement (123), insbesondere einem Durchgangsloch, zum Verbinden mit der hinteren Abdeckung (19), versehen wird, wobei das Metallband dem gekrümmten Profil der hinteren Abdeckung (19) folgt, wenn mit der hinteren Abdeckung (19) verbunden.

6. Vorrichtung (1, 1') gemäß einem der vorhergehenden Ansprüche, wobei der Kolben (11) einen Hauptkörper (21) umfasst, wobei eine erste Fläche (22) eine Druckkammer (17) mit dem ersten Ende (101) des Gehäuses (10) definiert und der Konstantkraft-Mechanismus (12, 12') zumindest teilweise auf einen inneren Bereich (23) des Hauptkörpers (21) des Kolbens (11) beschränkt ist.

7. Vorrichtung (1, 1') gemäß Anspruch 6, wobei der Hauptkörper (21) des Kolbens (11) eine zweite Fläche (24) umfasst, die zum manuellen Laden des Konstantkraft-Mechanismus (12, 12') verwendet wird.

8. Vorrichtung (1, 1') gemäß einem der vorhergehenden Ansprüche, wobei das Ventilsystem (13) an dem ersten Ende (101) des Längsgehäuse (10) positioniert ist.

9. Vorrichtung (1, 1') gemäß einem der vorhergehenden Ansprüche, wobei das Ventilsystem (13) zwei Einwegventile umfasst.

10. Vorrichtung (1, 1') gemäß einem der vorhergehenden Ansprüche, wobei das Ventilsystem (13) ein Abdeckelement (131, 131'), das an dem ersten Ende (101) des Längsgehäuse (10) befestigbar ist, und eine kreisförmige Membran (132), die zwischen dem Abdeckelement (131, 131') und dem ersten Ende (101) des Längsgehäuse (10) positioniert ist, umfasst.

11. Vorrichtung (1, 1') gemäß Anspruch 10, wobei das Abdeckelement (131, 131') eine erste Öffnung (133) für den Eintritt von Wundflüssigkeit und eine zweite Öffnung (134) für den Austritt von Luft umfasst.

12. Vorrichtung (1, 1') gemäß Anspruch 11, wobei das Längsgehäuse (10) einen vorderen Bereich (104) an dem ersten Ende (101) umfasst, wobei der vordere Bereich (104) ein Einlassloch (1011) an der ersten Öffnung (133) des Abdeckelements (131, 131') und ein Auslassloch (1012) an der zweiten Öffnung (134) des Abdeckelements (131, 131') umfasst.

13. Vorrichtung (1, 1') gemäß einem der Ansprüche 10 - 12, wobei die kreisförmige Membran (132) aus einem zentralen Scheibenelement (135) und einem umlaufenden Element (136), das konzentrisch zum Scheibenelement (135) ist und außerhalb davon positioniert ist, besteht, wobei das umlaufende Element (136) als eine Dichtung dient und zwischen dem Abdeckelement (131) und dem Längsgehäuse (10) befestigt ist.

14. Vorrichtung (1, 1') gemäß einem der Ansprüche 10 - 13, wobei das Längsgehäuse (10) einen vorderen Bereich (104) an dem ersten Ende (101) umfasst, wobei der vordere Bereich (104) ein spitz zulaufendes Profil (15) aufweist und zwei Flächen (151) umfasst, die eine Spitze definieren und die so konfiguriert sind, dass sie abwechselnd mit einer Hälfte der kreisförmigen Membran (132) nach dem Gleiten des Kolbens (11) im Inneren des Längsgehäuse (10) in Kontakt kommen.

15. Vorrichtung (1, 1') gemäß einem der Ansprüche 13 - 14, wobei der vordere Bereich (104) des Gehäuses eine äußere Mittelkante (153) umfasst, das Abdeckelement (131, 131') eine innere Mittelkante (138) umfasst und die zentrale Scheibe (135) der kreisförmigen Membran (132) in zwei Halbscheiben (1351, 1352) nach dem Zerdrücken der zentralen Scheibe (135) zwischen der äußeren Mittelkante (153) und der inneren Mittelkante (138) geteilt wird.

## Revendications

1. Dispositif à pression négative (1, 1') pour traiter une blessure et enlever le fluide de ladite blessure, le dispositif (1, 1') comprenant :
un logement longitudinal (10) ayant une première extrémité (101) configurée pour être connectée à la blessure et une seconde extrémité (102) opposée à la première extrémité (101), le logement longitudinal (10) étant une structure en une seule pièce, creuse et cylindrique,
un piston (11) configuré pour coulisser dans le logement (10) pour produire la pression négative,
un mécanisme à force constante (12, 12') configuré pour être chargé et mis en fonctionnement par le piston (11), ledit mécanisme à force constante (12, 12') étant fixé à la seconde extrémité (102) du logement longitudinal (10) et au piston coulissant (11) et étant disposé à l'intérieur du logement (10) et
un système de vanne (13) pour tirer l'air hors du logement (10) quand le piston (11) coulisse vers la première extrémité (101) du logement (10) et pour porter le fluide de blessure dans le logement (10) quand le piston (11) coulisse vers la seconde extrémité (102) du logement (10),
le mécanisme à force constante (12, 12') est entièrement contenu à l'intérieur du logement longitudinal (10) où le piston (11) coulisse et
**caractérisé en ce que**
la seconde extrémité (102) du logement longitudinal (10) comprend une ouverture arrière (14) ayant une taille telle qu'un ou plusieurs doigts d'une main d'un individu sont insérables à l'intérieur pour faire coulisser manuellement le piston (11) et charger le mécanisme à force constante (12, 12').

2. Dispositif (1') selon la revendication 1, dans lequel le logement (10) comprend une couverture arrière (19) incurvée à la seconde extrémité (102) recouvrant partiellement le dos du dispositif (1').

3. Dispositif (1') selon la revendication 2, dans lequel l'élément de couverture (19) comprend un élément de fixation (191) pour fixer le mécanisme à force constante (12') au logement (10).

4. Dispositif (1, 1') selon l'une des revendications précédentes, dans lequel le mécanisme à force constante (12, 12') comprend au moins un ressort à force constante (121) fixé au piston (11) de manière laminaire qui peut être activé par la pression exercée par un ou plusieurs doigts d'un simple utilisateur.

5. Dispositif (1') selon les revendications 2, 3 et 4, dans lequel le mécanisme à force constante (12') comprend un simple ressort à force constante monté sur un support cylindrique (122), dans lequel le ressort est formé par une bande métallique qui peut être enroulée autour dudit support cylindrique (122), dans lequel une extrémité de la bande est fixée au support cylindrique (122) et l'autre extrémité de la bande est fournie avec un élément de connexion (123), en particulier un trou, pour connecter à la couverture arrière (19), la bande métallique suivant le profil incurvé de la couverture arrière (19) quand elle est connectée à ladite couverture arrière (19).

6. Dispositif (1, 1') selon l'une des revendications précédentes, dans lequel le piston (11) comprend un corps principal (21) avec une première surface (22) définissant une chambre de pression (17) avec la première extrémité (101) du logement (10) et le mécanisme à force constante (12, 12') est confiné, au moins partiellement, dans une zone interne (23) dudit corps principal (21) du piston (11).

7. Dispositif (1, 1') selon la revendication 6, dans lequel le corps principal (21) du piston (11) comprend une seconde surface (24) utilisée pour charger manuellement le mécanisme à force constante (12, 12').

8. Dispositif (1, 1') selon l'une des revendications précédentes, dans lequel le système de vanne (13) est placé à la première extrémité (101) du logement longitudinal (10).

9. Dispositif (1, 1') selon l'une des revendications précédentes, dans lequel le système de vanne (13) comprend deux vannes à une voie.

10. Dispositif (1, 1') selon l'une des revendications précédentes, dans lequel le système de vanne (13) comprend un élément de couverture (131, 131') fixable à la première extrémité (101) du logement longitudinal (10) et une membrane circulaire (132) positionnée entre l'élément de couverture (131, 131') et la première extrémité (101) du logement longitudinal (10).

11. Dispositif (1, 1') selon la revendication 10, dans lequel l'élément de couverture (131, 131') comprend une première ouverture (133) pour l'entrée du fluide de blessure et une seconde ouverture (134) pour la sortie de l'air.

12. Dispositif (1, 1') selon la revendication 11, dans lequel le logement longitudinal (10) comprend une portion frontale (104) à la première extrémité (101), ladite portion frontale (104) comprenant un trou d'entrée (1011) à la première ouverture (133) de l'élément de couverture (131, 131') et un trou de sortie (1012) à la seconde ouverture (134) de l'élément de couverture (131, 131').

13. Dispositif (1, 1') selon l'une des revendications 10 à 12, dans lequel la membrane circulaire (132) est constituée d'un élément de disque central (135) et d'un élément circonférentiel (136) concentrique avec l'élément de disque (135) et positionné de manière externe à celui-ci, l'élément circonférentiel (136) agissant comme un joint et étant fixé entre l'élément de couverture (131) et le logement longitudinal (10).

14. Dispositif (1, 1') selon l'une des revendications 10 à 13, dans lequel le logement longitudinal (10) comprend une partie frontale (104) à la première extrémité (101), ladite partie frontale (104) ayant un profil pointé (15) et comprenant deux surfaces (151) définissant une pointe et qui sont configurées pour être en alternance en contact avec une moitié de la membrane circulaire (132) suivant le coulissement du piston (11) à l'intérieur du logement longitudinal (10).

15. Dispositif (1, 1') selon l'une des revendications 13 à 14, dans lequel la portion frontale (104) du logement comprend un bord central externe (153), l'élément de couverture (131, 131') comprend un bord central interne (138) et le disque central (135) de la membrane circulaire (132) est divisé en deux demi-disques (1351, 1352) suivant le fractionnement dudit disque central (135) entre ledit bord central externe (153) et ledit bord central interne (138).
